# EUROPEAN PATENT APPLICATION

(11) **EP 4 098 738 A1**
(43) Date of publication of application: **07.12.2022**
(21) Application number: 21846360.2
(22) Date of filing: 26.01.2021
(51) Int. Cl.: C12M 3/02, C12M 1/02

(54) **STIRRING SYSTEM FOR BIOREACTOR**

(30) Priority: 23.07.2020 CN 202010717020
(71) Applicant: Alit Biotech (Shanghai) Co., Ltd., Shanghai 201615 (CN)
(72) Inventor: LIU, Yu, Shanghai 201613 (CN); CHEN, Rui, Shanghai 201613 (CN)
(74) Representative: Wang, Bo
(86) International application number: PCT/CN2021/073802
(87) International publication number: WO 2022/016846

(57) **Abstract**

The present disclosure relates to a stirring system for a bioreactor. The bioreactor comprises a tank for containing a mixture of cells and a liquid, wherein the stirring system comprises: a central shaft; and a multi-layer paddle component, the multi-layer paddle component is immersed in the mixture and includes an upper paddle component, an intermediate paddle component and a bottom paddle component arranged around a central shaft. The bottom paddle component is arranged close to the bottom of the tank and is driven by a driver outside the tank in a contactless manner, and the intermediate paddle component is arranged between the upper paddle component and the bottom paddle component. The bottom paddle component also includes a helical paddle. The stirring system according to the present disclosure can not only provide sufficient lifting force at a low speed to make the microcarriers keep suspended, but also reduce the damage of the shear force to animal cells as much as possible.

## Description

### TECHNICAL FIELD

The present disclosure generally relates to the field of bioreactors. More specifically, the present disclosure relates to a stirring system for bioreactors.

### BACKGROUND

In the traditional stirring bioreactor design, especially in the animal cell reactor, the design of the stirring paddle is usually single-layer, double-layer or multi-layer, and the distance between the bottom stirring paddle and the bottom of the tank is the diameter or 1/2 of the diameter of a blade. At present, the application scenario of animal cell reactors has gradually developed from CHO cells to T cells and stem cells. CHO cells have been genetically modified and domesticated in the laboratory, and are relatively suitable for suspension culture. However, stem cells and T cells, especially primary cells in the field of cell therapy, are difficult to adapt to suspension culture, which shows that they are particularly sensitive to fluid hydraulic shear and bubbles during reactor expansion. This situation also occurs in the process of the transformation from other adherent cell culture mode to suspension culture mode.

Microcarriers are a way to help adherent cells suspend. By attaching microcarriers, adherent cells or cells that are difficult to adapt to suspension culture can better adapt to suspension culture. The diameter of microcarriers usually ranges from tens of microns to millimeters, and they have different shapes. For example, microcarriers can include liquid microcarriers, macroporous gelatin microcarriers, polystyrene microcarriers, pHEMA microcarriers, chitin microcarriers, polyurethane foam microcarriers, alginate gel microcarriers, magnetic microcarriers, etc.

The diameter of microcarriers is larger and the density is usually slightly larger than that of water, so the sedimentation coefficient is higher. In order to keep the microcarrier suspended, the rotating speed of the agitator needs to be increased above the predetermined value. However, animal cells have no cell wall and are sensitive to shear force. The increase of impeller speed will cause damage to animal cells. Therefore, there is a contradiction between maximizing the suspension time and minimizing the shear force in the design of the impeller. The traditional design scheme of stirring paddles of bioreactor is more suitable for the environment with uniform mixing under high-speed process conditions. For stem cells, T cells or other adherent cells and microcarrier processes, it is necessary to provide a new stirring system, which can not only provide enough lift at low speed to make the microcarrier suspend continuously, but also reduce the damage of shear force to animal cells.

### SUMMARY

One of the purposes of the present disclosure is to provide a stirring system that can overcome at least one defect in the prior art.

The first aspect of the present disclosure relates to a stirring system for a bioreactor, which comprises a tank for containing a mixture of cells and liquids, which is characterized in that the stirring system comprises:

a central shaft; and a multi-layer paddle component, wherein the multi-layer paddle component is immersed in the mixture and includes an upper paddle component, an intermediate paddle component and a bottom paddle component arranged around the central shaft, the bottom paddle component being arranged close to the bottom of the tank and being driven by a driver outside the tank in a contactless manner, and the intermediate paddle component being arranged between the upper paddle component and the bottom paddle component and also including a helical paddle.

In some embodiments, the bottom paddle component includes a hub part and two or more inclined paddle paddles protruding from the hub part.

In some embodiments, a bottom end of the central shaft is fixed to the top wall of the hub, and a top end of the central shaft is rotatably supported by the top cover of the tank.

In some embodiments, the hub part is provided with a pivot aperture in the center of its bottom wall, so that it is supported on the center column at the bottom of the tank and rotatable around the center column.

In some embodiments, the driver includes a magnetic stirrer, and the hub includes a chamber containing a magnet configured to be magnetically coupled to the magnetic stirrer.

In some embodiments, the magnetic stirrer is configured to drive the multilayer paddle component to rotate about a central shaft in a contactless manner.

In some embodiments, the hub is a flat plate, and a cross section of the hub is circular, elliptical, or polygonal with a straight side or a curved side.

In some embodiments, the two or more inclined paddle paddles are configured to be fixed to the side wall of the hub and extend from the side wall along a radial direction.

In some embodiments, the height of the skewed paddle is less than or equal to the height of the hub.

In some embodiments, the structures of the inclined blades are formed by scanning a curve surface on a curve of a side wall of the hub part, and the curve being a curve defined by a mathematical formula of a linear equation, a mathematical formula of a quadratic equation, or a mathematical formula of a cubic equation.

In some embodiments, the structures of the inclined blades are formed by moving a straight line perpendicular to an outer surface of the side wall of the hub from one end of the curve to the other end of the curve along the curve on the side wall of the hub part.

In some embodiments, there is a gap between adjacent inclined paddle paddles in the circumferential direction.

In some embodiments, the bottom paddle component is 2 mm-10 mm from the bottom of the tank.

In some embodiments, the intermediate paddle component includes one or more helical paddles axially distributed along the central shaft.

In some embodiments, each helical paddle has a hub part and two or more helical blades surrounding the hub part.

In some embodiments, structures of the helical blades are formed by a curved surface scanning along a spiral line on a side wall of the hub part.

In some embodiments, the structures of the helical blades are formed by moving a straight line perpendicular to an outer surface of the side wall of the hub part along the spiral line on the side wall of the hub part from one end of the spiral line to the other end of the spiral line.

In some embodiments, the diameter of the helical blade of the helical paddle is 0.4-0.6 times the inner diameter of the tank.

In some embodiments, the guiding cylinder is arranged in the tank and around the helical paddle, and the guiding cylinder is configured to make the upward flowing mixture in a unidirectional laminar flow state.

In some embodiments, the guiding cylinder is configured as a cell separation apparatus, which has a filter element for filtering cells in its radial interior.

In some embodiments, the helical paddle is arranged at the axial middle part inside the cell separation apparatus and is radially separated from the filter element by a small radial clearance.

In some embodiments, the radial clearance ranges from 0.1 mm to 10 mm.

In some embodiments, the gas distributor is arranged between the bottom paddle component and the intermediate paddle component, and is configured to provide gas in the form of bubbles to the cells in the tank.

In some embodiments, the upper paddle component includes one or more skewed paddles axially distributed along the central shaft.

In some embodiments, the diameter of the paddless of the paddles is 0.4-0.6 times the inner diameter of the tank.

In some embodiments, the tilt angle of the paddles of the inclined blade is between 30° and 60°.

In some embodiments, each inclined blade includes 2 to 6 paddles.

In some embodiments, the paddles of each inclined blade do not overlap or overlap slightly in the circumferential direction.

In some embodiments, the upper paddle component is arranged at a distance of 1.0-1.5 times the diameter of the inclined blade from the intermediate paddle component.

In some embodiments, the axial distance between the upper paddle component and the intermediate paddle component is set to be greater than the axial distance between the intermediate paddle component and the bottom paddle component.

In some embodiments, a bioreactor is a bioreactor for stem cells and T cells.

Another aspect of the present disclosure relates to a bioreactor, which comprises the above-mentioned stirring system.

Other features and advantages of the subject technology of the present disclosure will be described in the following description, and will be apparent in part from the description, or can be learned by practicing the subject technology of the present disclosure. The advantages of the subject technology of the present disclosure will be realized and obtained through the structure specially pointed out in the written description, the claims and the accompanying drawings.

It should be understood that the foregoing general description and the following detailed description are both exemplary and illustrative, and are intended to provide further description of the subject technology of the present disclosure that requires protection.

### BRIEF DESCRIPTION OF THE DRAWINGS

After reading the following specific embodiments in combination with the accompanying drawings, various aspects of the present disclosure will be better understood. In the accompanying drawings:
FIG. 1 is a schematic diagram of the use environment of the stirring system according to the embodiment of the present disclosure;
FIGs. 2A and 2B are perspective and front views of a stirring system according to an embodiment of the present disclosure;
FIGs. 3A and 3B are perspective and front views of the bottom paddle component of the stirring system shown in FIG. 2;
FIG. 4A is a front view of the intermediate paddle component of the stirring system shown in FIG. 2; FIG. 4B is a multiple variant design of the intermediate paddle component;
FIGs. 5A and 5B are the flow field velocity vector diagrams of the stirring system shown in FIG. 2; and
FIG. 6 shows the experimental results of the performance comparison between the stirring system shown in FIG. 2 and the traditional double-layer elephant ear paddle.

### DETAILED DESCRIPTION

The present disclosure will be described below with reference to the accompanying drawings, which show several embodiments of the present disclosure. However, it should be understood that the present disclosure can be presented in a variety of different ways, and is not limited to the embodiments described below; In fact, the embodiments described below are intended to make the present disclosure of this disclosure more complete and fully explain the scope of protection of the present disclosure to those skilled in the art. It should also be understood that the embodiments disclosed herein can be combined in various ways to provide more additional embodiments.

It should be understood that in all the drawings, the same reference numerals represent the same elements. In the drawings, the dimensions of some features can be deformed for clarity.

It should be understood that the terms in the specification are only used to describe specific embodiments and are not intended to limit the present disclosure. Unless otherwise defined, all terms (including technical terms and scientific terms) used in the specification have the meanings commonly understood by those skilled in the art. For simplicity and/or clarity, well-known functions or structures may not be described in detail.

The singular forms "one", "said" and "this" used in the instructions include the plural unless clearly indicated. The terms "including", "including" and "containing" used in the specification indicate the existence of the claimed feature, but do not exclude the existence of one or more other features. The term "and / or" used in the specification includes any and all combinations of one or more of the relevant listed items. The terms "between X and Y" and "between approximately X and Y" used in the instructions should be interpreted to include X and y. The term "between about X and Y" used in this manual means "between about X and about y", and the term "from about X to Y" used in this manual means "from about X to about y".

In the present disclosure, when an element is said to be "on", "attached" to another element, "connected" to another element, "coupled" to another element, or "in contact" with another element, the element can be directly on another element, attached to another element, connected to another element, connected to another element, or in contact with another element, or there can be intermediate elements. In contrast, when an element is said to be "directly" on another element, "directly attached" to another element, "directly connected" to another element, "directly coupled" to another element or "directly in contact" with another element, there will be no intermediate element. In the present disclosure, one feature is arranged to be "adjacent" to another feature, which can mean that one feature has a part overlapping with the adjacent feature or a part above or below the adjacent feature.

In the present disclosure, the spatial relationship terms such as "up", "down", "left", "right", "front", "back", "high", "low" and so on can explain the relationship between one feature and another feature in the drawings. It should be understood that the term spatial relationship includes different orientations of the device in use or operation in addition to the orientations shown in the accompanying drawings. For example, when the device in the drawings is reversed, the feature originally described as "below" other features can be described as "above" other features at this time. The device can also be oriented in other ways (rotate 90 degrees or in other directions), at which time the relative spatial relationship will be interpreted accordingly.

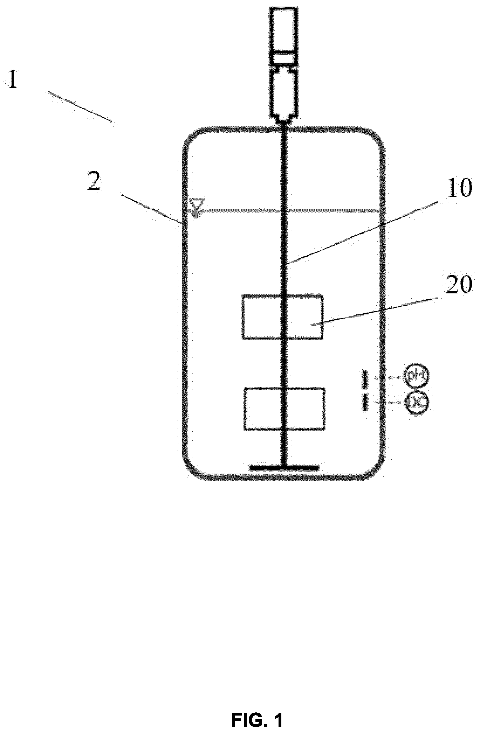

FIG. 1 shows a schematic diagram of a stirring system 1 according to an embodiment of the present disclosure. The stirring system 1 is applicable to various bioreactors, especially bioreactors of animal cells (such as stem cells and T cells). The bioreactor includes a tank 2 to contain a mixture of cells, microcarriers and culture media, or a mixture of cells, microcarriers and other liquids. As shown in the figure, the stirring system 1 includes a central shaft 10 and a multi-layer paddle component 20 arranged around the central shaft 10. The multi-layer paddle component 20 is immersed in a mixture of cells, microcarriers, and culture media (or other liquids). The multi-layer paddle component 20 may form a large upward thrust on the mixture at a lower speed to form an upstream flow field in the tank 2, thereby improving the suspension capacity of the microcarrier and the cells attached to the microcarrier.

The multi-layer paddle component 66 may be a three-layer paddle component, a four-layer paddle component, or a paddle component including more layers. Each layer of the paddle component can be set to be the same or different from each other. Taking the three-layer paddle component as an example, the structure of the multi-layer paddle component 66 of the stirring system 60 according to the embodiment of the present disclosure is described below.

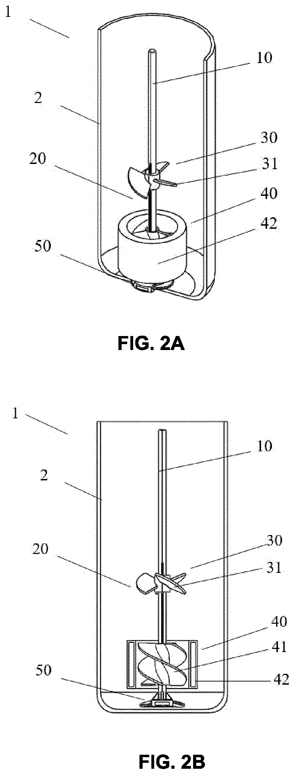

FIGs. 2A and 2B show a perspective view and a front view of the stirring system 1, respectively. As shown in the figure, the multi-layer paddle component 20 includes an upper paddle component 30, an intermediate paddle component 40, and a bottom paddle component 50. The upper paddle component 30, the intermediate paddle component 40, and the bottom paddle component 50 are arranged around the central shaft 10 and are immersed in a mixture of cells, microcarriers, and culture media (or other liquids). The upper paddle component 30 is arranged at the upper part of the tank 2, the bottom paddle component 50 is arranged close to the bottom of the tank 2, and the intermediate paddle component 40 is arranged between the upper paddle component 30 and the bottom paddle component 50. The axial distance between the upper paddle component 30 and the intermediate paddle component 40 and the axial distance between the intermediate paddle component 40 and the bottom paddle component 50 can be set to be the same or different from each other. In some embodiments, the axial distance between the upper paddle component 30 and the intermediate paddle component 40 may be set to be greater than the axial distance between the intermediate paddle component 40 and the bottom paddle component 50.

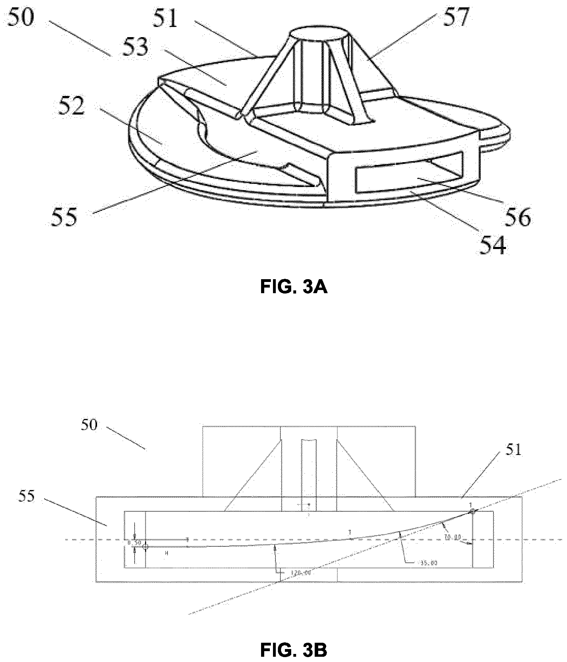

FIGs. 3A and 3B show a perspective view and a front view of the bottom paddle component 50, respectively. As shown in the figure, the bottom paddle component 50 is used to transport particles such as microcarriers and/or cells at the bottom of the tank 2 to the upper paddle component. The bottom paddle component 50 includes a hub part 51 and two or more inclined blades 52 extending from the hub part 51 along a radial direction. The hub part 51 is in a flat disk shape, and the cross section can be circular, elliptical, triangular, quadrilateral, or other polygons, which can include straight sides or curved sides.

The hub part 51 includes a top wall 53, a bottom wall 54, and a side wall 55 connecting the top wall 53 and the bottom wall 54. The top wall 53, the bottom wall 54, and the side wall 55 surround the middle cavity 56 of the hub part 51. The center of the top wall 53 of the hub part 51 is fixed to the bottom end of the central shaft 10, so that the hub part 51 can rotate together with the central shaft 10, and the top end of the central shaft 10 can be rotatably supported by the top cover of the tank 2. In some embodiments, the connecting portion between the hub part 51 and the central shaft 10 may be reinforced by a reinforcing rib 57. The bottom wall 54 of the hub part 51 is provided with a pivot aperture in its center, so that it can be supported on the center column at the bottom of the tank 2 and rotate around the center column at the bottom of the tank 2. The middle cavity 56 of the hub part 51 houses a magnet. The magnet can be magnetically coupled with a driver (such as a magnetic stirrer) outside the tank 2, so that the driver can drive the entire multi-layer paddle component 20 to rotate around the central shaft 10. The combination of magnet and driver provides driving power for the stirring system 1 in a contactless way, avoiding the problems of liquid leakage or microbial pollution in the contact drive mechanism arrangement scheme (such as the scheme of motor driving central shaft, etc.).

The inclined blade 52 is fixed to the side wall 55 of the hub part 51 and extends radially outward from the side wall 55. The number of inclined blades 52 can be set to 2, 3, or more. The inclined blades 52 may be arranged to be evenly distributed in a circumferential direction around the hub part 51. The inclined blades 52 is structured by scanning a curve surface on a curve of a side wall 55 of the hub part 51. Specifically, a straight line (corresponding to the width of the blade) perpendicular to the outer surface of the side wall 55 of the hub part 51 is moved along the curve drawn on the side wall 55 of the hub part 51 from one end of the curve to the other end of the curve to form the inclined blades 52. FIG. 3B shows a curve drawing method of inclined blades 52. The method is to ensure that the lower starting point on the left side of the curve is located in the lower part of the hub part 51, and the upper end point on the right side of the curve is located in the upper part of the hub part 51. The curve at the lower starting point is tangent to the horizontal line, and the curve at the upper end point maintains a certain angle with the horizontal line; The natural transition of the curve is ensured through the control of 2-3 points between the lower starting point and the upper ending point. The inclined blades 52 can also use other curve drawing methods, such as the linear drawing method, the spiral line drawing method, or any other linear equation, the secondary equation, and the three equations.

Since the inclined blade 92 is close to the bottom of the tank 62, the dead zone (i.e., an area in which the mixture stops flowing) below the inclined blades 92 can be reduced as much as possible by reducing the paddle area of the inclined blades 92. Alternatively, a part of the volume may be left between adjacent inclined blades 92, or there may be a gap in the circumferential direction, so as to maintain the mixing effect and reduce the dead zone under the paddle as much as possible.

The bottom paddle component 50 adopts a unique combination of the hub part 51 and the inclined blades 52 fixed to the side wall of the hub part 51. The hub part 51 adopts a flat disc body, and the height of the inclined blades 52 is less than or equal to the height of the hub part 51, so the height of the whole combination may be set to be small. The bottom paddle component 50 can be located as low as possible in the bioreactor tank 2 to provide power in the bottom environment of the tank 2 to transport the microcarriers or cells at the bottom of the tank 2 to the upper paddle component, thereby strengthening the continuous suspension capacity of the microcarriers or cells at the bottom of the tank 2.

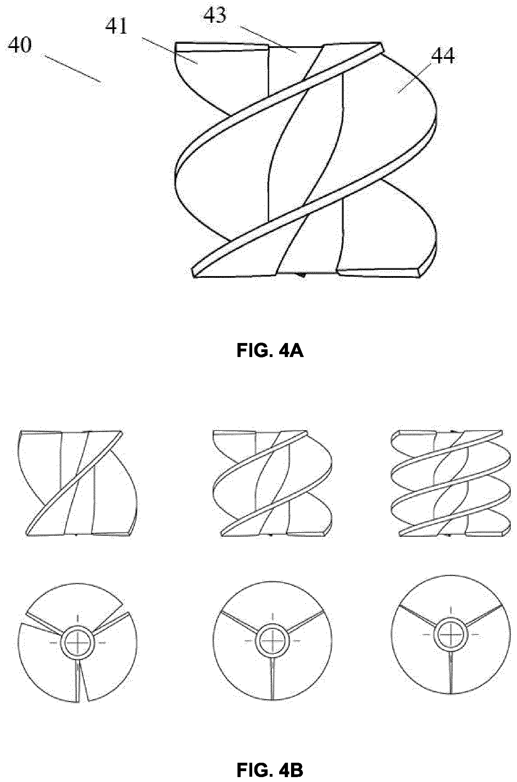

FIG. 4A shows a front view of the intermediate paddle component 40. As shown in the figure, the intermediate paddle component 40 is used to directionally push particles such as microcarriers or cells stirred by the bottom paddle component 50 into the ascending channel. The intermediate paddle component 40 includes a helical paddle 41 surrounding the central shaft 10, and the helical blade 41 has a cylindrical hub part 43 and helical blades 44 surrounding the hub part 43.

The number of helical blades 44 can be set to 2, 3, or more. The helical blades 44 may be arranged to be uniformly distributed in a circumferential direction around the central shaft 10. The helical blade 44 is formed by scanning the curved surface of the spiral line drawn on the side wall of the hub part 43; Specifically, the helical blade 44 is formed by moving a straight line (corresponding to the width of the paddle) perpendicular to the outer surface of the side wall of the hub part 43 along the helix drawn on the side wall of the hub part 43 from one end of the helix to the other end of the helix. The spiral takes the cross-sectional circle of the hub part 43 as the diameter and the height of the hub part 43 as the height, and the variable pitch and number of turns of the spiral ensure that the helical blades 44 and the hub part 43 are at the same height. As shown in FIG. 4B, the pitch and number of revolutions of each helical blade 44 can be determined according to actual needs, and the difference it brings is the difference between speed and pumping flow. In some embodiments, the intermediate paddle component 40 may also include a plurality of helical paddle 41, and these helical paddles 41 may be axially distributed along the central shaft 10.

Returning to FIGs. 2A and 2B, a guiding cylinder 42 can be arranged in the tank 2, and the guiding cylinder 42 surrounds the helical paddle 41. The guiding cylinder 42 is used to guide the upward flowing mixture, so that the upward flowing mixture will not converge with the reflux mixture, so as to ensure that the upward flowing mixture is in a unidirectional laminar flow state, which is very important for microcarriers or single suspended cells.

The guiding cylinder 42 is used to guide the upward flowing mixture, so that the upward flowing mixture will not converge with the reflux mixture, so as to ensure that the upward flowing mixture is in a unidirectional laminar flow state, which is very important for microcarriers or single suspended cells. The cell separation apparatus is in the shape of a hollow cylinder, and has a filter element for filtering cells in its radial interior. The helical blade 41 can be arranged in the axial middle part inside the cell separation apparatus, and is radially separated from the filter element by a small gap. Due to the narrow radial gap between the helical paddle 41 and the filter element, the clockwise or counterclockwise rotation of the helical paddle 41 will produce a pulse vortex in the narrow radial gap, which can remove the cells attached to the filter element. The radial clearance between the helical paddle 41 and the filter element ranges from 0.1 mm to 10 mm, and the size is related to the volume and diameter of the tank 2. If the radial clearance increases, the damage caused by shear force to cells decreases, but the eddy energy formed decreases, so the ability to remove cell blockers on the filter element decreases; On the contrary, if the radial gap is reduced, the shear force damage to cells is significantly increased, but the energy of vortex formation is increased, so the ability to remove cell blockers on the filter element is increased. Therefore, the determination of the gap size is one of the core parameters of the stirring system 1. Depending on the type, shape, diameter and density of cells in the bioreactor, the helical paddle 41 can have a variety of deformations, such as the increase of paddle pitch, the increase of the number of paddles, and so on. These increases will increase the flow of the filter element of the cell separation apparatus and change the fluid velocity, thus affecting the filtration flow.

In some embodiments, the gas distributor may be arranged between the bottom paddle component 50 and the intermediate paddle component 40 and used to provide gas in the form of bubbles to the cells in the tank 2. The design of the helical blade 41 of the intermediate paddle component 40 and the guiding cylinder 42 enhances the mass transfer of bubbles in the guiding cylinder 42, and bubbles can provide assistance for the suspension of particles such as microcarriers.

FIG. 2A and 2B also show a perspective view and a front view of the upper paddle component 30. As shown in the figure, the upper paddle component 30 is arranged on the upper part of the liquid of the tank 2 and is mainly used to provide mixing. The upper paddle component 30 may include an inclined paddle 31 having a hub part and paddles surrounding the hub part. The inclined paddle 31 generally has three blades, and can also have two to six blades. In the top view, the blades of the inclined paddle 31 do not overlap or overlap a little, and the coverage area is the circle of the entire diameter, and the center of the paddle can be located on the hub part. The inclination angle of the blade of the inclined paddle 31 is generally 30°, 45° or 60°. The sharp angle of paddles of inclined paddle 31 is generally chamfered to reduce the shear force or the extreme value of speed. Depending on the angle of the paddle in the clockwise direction, the inclined paddle 31 may drive the fluid upward or downward. In some embodiments, the upper paddle component 30 may also include a plurality of inclined paddle 31, and these inclined paddles 31 may be axially distributed along the central shaft 10.

Compared with the traditional stirring system of the bioreactor, the bottom paddle component 50 and the intermediate paddle component 40 of the stirring system 1 according to the present disclosure are closer to the bottom of the tank 2. The bottom paddle component 50 is 2 mm-10 mm away from the bottom of the tank 2, while the intermediate paddle component 40 and the guiding cylinder 42 are close to the bottom paddle component 50, so that the suspended particles can be effectively sucked into the middle layer guide area. This design can effectively ensure that the stirring system in the bioreactor may also keep particles such as microcarriers or cells suspended at a low speed. The diameters of the upper paddle component 30 and the intermediate paddle component 40 are set to be approximately the same, and are 0.4-0.6 times the inner diameter of the tank 2. The upper paddle component 30 is usually designed to be approximately 1.0-1.5 times the diameter from the intermediate paddle component 40, which mainly provides mixing.

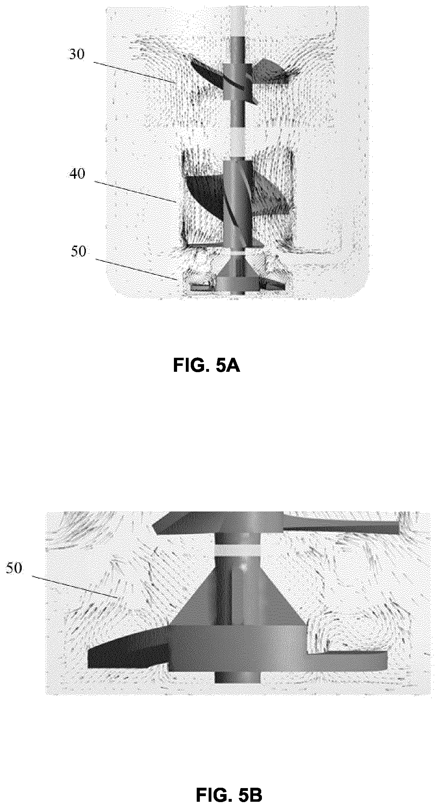

FIGs. 5A and 5B show the velocity vector diagram of the flow field driven by the computer simulated stirring system 1. It can be seen from FIG. 5A that the stirring system 1 forms a good liquid circulation in the tank 2 of the bioreactor. The bottom paddle component 50 and the intermediate paddle component 40 regulate the flow state of the liquid to form a laminar flow in a single direction; the upper paddle component 30 has a good mixing effect on the liquid lifted by the lower paddle component. As can be seen from FIG. 5B, the bottom paddle component 50 forms an upward flowing fluid state, which may support the continuous suspension of the microcarrier, and has a good effect on improving the flow field at the bottom of tank 2.

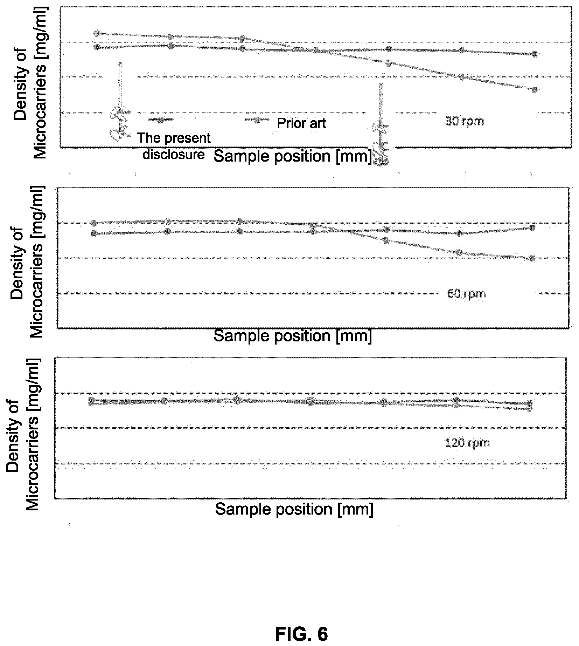

FIG. 6 shows the performance comparison experiment of the stirring system 1 according to the present disclosure and the traditional double-layer Elephant Ear paddle at different speeds. The slurry diameter ratio of stirring system 1 is consistent with that of traditional double-layer elephant ear paddles, both of which are 0.45; the concentration of the microcarrier is obtained by sampling along the axial direction of the tank 2. The abscissa in FIG. 6 represents the sampling position along the axial direction of tank 2, and the ordinate represents the concentration of microcarriers. It can be seen from the figure that the microcarrier suspension capacity of the traditional double-layer elephant ear paddles at low speed is not ideal, and the minimum speed for suspending the microcarrier is between 90 rpm and 120 rpm, while the minimum speed can be effectively reduced to 30 rpm according to the stirring system 1 of the present disclosure. Therefore, according to the disclosed stirring system 1, it can provide sufficient lift at a low speed to continuously suspend microcarriers, but also reduce the damage to animal cells as much as possible.

In addition, the stirring system 1 according to the present disclosure can effectively remove the cells attached to the filter element and is not easy to block the filter element, thereby ensuring the long-term use of the cell separation apparatus.

Although the exemplary embodiments of the present disclosure have been described, those skilled in the art should understand that various changes and changes can be made to the exemplary embodiments of the present disclosure without departing from the spirit and scope of the present disclosure in essence. Therefore, all changes and changes are included in the scope of protection of the present disclosure as defined in the claims. The present disclosure is defined by additional claims, and equivalents of these claims are also included.

## Claims

1. A stirring system for a bioreactor comprising a tank used for containing a mixture of cells and liquids, wherein the stirring system comprises:
a central shaft; and
a multi-layer paddle component, wherein
the multi-layer paddle component is immersed in the mixture and includes an upper paddle component, an intermediate paddle component and a bottom paddle component arranged around the central shaft, the bottom paddle component being arranged close to the bottom of the tank and being driven by a driver outside the tank in a contactless manner, the intermediate paddle component being arranged between the upper paddle component, and the intermediate paddle component including a helical paddle.

2. The stirring system according to claim 1, wherein the bottom paddle component comprises a hub part and two or more inclined blades protruding from the hub part.

3. The stirring system according to claim 2, wherein a bottom end of the central shaft is fixed to a top wall of the hub, and a top end of the central shaft is rotatably supported by a top cover of the tank.

4. The stirring system according to claim 2, wherein the hub part is provided with a pivot aperture in a center of its bottom wall, thereby being supported by a center column at the bottom of the tank and rotatable around the center column.

5. The stirring system according to claim 2, wherein the driver comprises a magnetic stirrer, and the hub part comprises a chamber containing a magnet configured to be magnetically coupled with the magnetic stirrer.

6. The stirring system according to claim 5, wherein the magnetic stirrer is configured to drive the multi-layer paddle component to rotate around the central shaft in a contactless manner.

7. The stirring system according to claim 2, wherein the hub part is a flat plate, and a cross section of the hub part is circular, elliptical, or polygonal with a straight side or a curved side.

8. The stirring system according to claim 2 , wherein the two or more inclined blades are configured to be fixed to a side wall of the hub part, and extending from the side wall along a radial direction.

9. The stirring system according to claim 2, wherein a height of the inclined blades is less than or equal to a height of the hub part.

10. The stirring system according to claim 2, wherein the structures of the inclined blades are formed by a curved surface scanning on a curve of a side wall of the hub part, and the curve being a curve defined by a mathematical formula of a linear equation, a mathematical formula of a quadratic equation, or a mathematical formula of a cubic equation.

11. The stirring system according to claim 10, wherein the structures of the inclined blades are formed by moving a straight line perpendicular to an outer surface of the side wall of the hub from one end of the curve to the other end of the curve along the curve on the side wall of the hub part.

12. The stirring system according to claim 2, wherein there is a gap between adjacent inclined blades in the circumferential direction.

13. The stirring system according to claim 1, wherein a distance between the bottom paddle component and the bottom of the tank is 2 mm-10 mm.

14. the stirring system according to claim 1, wherein the intermediate paddle component comprises one or more helical paddles axially distributed along the central shaft.

15. The stirring system according to claim 14, wherein each of the helical paddle has a hub part and two or more helical blades surrounding the hub part.

16. The stirring system according to claim 15, wherein the structures of the helical blades are formed by a curved surface scanning along a spiral line on a side wall of the hub part.

17. The stirring system according to claim 16, wherein the structures of the helical blades are formed by moving a straight line perpendicular to an outer surface of the side wall of the hub part along the spiral line on the side wall of the hub part from one end of the spiral line to the other end of the spiral line.

18. The stirring system according to claim 15, wherein the helical blades of the helical paddle is 0.4-0.6 times an inner diameter of the tank.

19. The stirring system according to claim 14, wherein a guiding cylinder is arranged in the tank and around the helical paddle, and the guiding cylinder is configured to cause the upward flowing mixture to be in a unidirectional laminar flow state.

20. The stirring system according to claim 19, wherein the guiding cylinder is configured as a cell separation apparatus having a filter element for filtering cells in radial interior.

21. The stirring system according to claim 20, wherein the helical paddle is arranged in an axial middle part inside the cell separation apparatus, and radially separated from the filter element by a small radial gap.

22. The stirring system according to claim 21, wherein the radical gap ranges from 0.1 mm to 10 mm.

23. The stirring system according to claim 1, wherein a gas distribution device is arranged between the bottom paddle component and the intermediate paddle component, and is configured to provide gas to the cells in the tank in the form of bubbles.

24. The stirring system according to claim 1, wherein the upper paddle component includes one or more inclined paddles distributed along the center shaft.

25. The stirring system according to claim 24, wherein a diameter of an inclined blade of the inclined paddle is 0.4-0.6 times of the inner diameter of the tank.

26. The stirring system according to claim 24, wherein an inclination angle of the inclined blade is between 30° and 60°.

27. The stirring system according to claim 24, wherein each inclined paddle includes 2 to 6 blades.

28. The stirring system according to claim 24, wherein the inclined blades of each inclined paddle do not overlap in the circumferential direction, or overlap by a little.

29. The stirring system according to claim 24, wherein the upper paddle component is set at a distance of 1.0-1.5 times the diameter of the inclined blades from the intermediate paddle component.

30. The stirring system of any of claim 1-29, wherein an axial distance between the upper paddle component and the intermediate paddle component is set to be greater than an axial distance between the intermediate paddle component and the bottom paddle component.

31. The stirring system of any one of claims 1-29, wherein the bioreactor is a bioreactor for stem cells and T cells.

32. A bioreactor, comprising the stirring system according to any one of claims 1-31.
